(19) Europäisches Patentamt
European Patent Office
Office européen des brevets

(11) Publication number: **0 187 382 B1**

(12) # EUROPEAN PATENT SPECIFICATION

(45) Date of publication of patent specification: **24.07.91**

(51) Int. Cl.5: **C12N 15/55**, C12N 15/56, C12N 1/21, //(C12N1/21, C12R1:19)

(21) Application number: **85116611.6**

(22) Date of filing: **27.12.85**

(54) **Plasmid, method for construction of the same, microorganism carrying the plasmid and method for cultivation of the microorganism.**

(30) Priority: **28.12.84 JP 278681/84**

(43) Date of publication of application:
**16.07.86 Bulletin 86/29**

(45) Publication of the grant of the patent:
**24.07.91 Bulletin 91/30**

(84) Designated Contracting States:
**DE FR NL**

(56) References cited:
**EP-A- 0 121 138**

**AGRIC. BIOL. CHEM., vol. 50, no. 4, 1986, pages 1067,1068; C. KATO et al.: "Extracellular production of xylanase"**

**CHEMICAL ABSTRACTS, vol. 86, no. 19, 9th May 1977, page 264, abstract no. 136179y, Columbus, Ohio, US; R.L. RODRIGUEZ et al.: "Construction and characterization of cloning vehicles", & ICN-UCLA SYMP. MOL. CELL. BIOL. 1976, 5(Mol. Mech. Control Gene Expression), 471-7**

(73) Proprietor: **RIKAGAKU KENKYUSHO**
**2-1 Hirosawa**
**Wako-shi Saitama-ken(JP)**

(72) Inventor: **Horikoshi, Koki**
**No. 4-39-8, Sakuradai Nerima-ku**
**Tokyo(JP)**

(74) Representative: **Vossius & Partner**
**Siebertstrasse 4 P.O. Box 86 07 67**
**W-8000 München 86(DE)**

**EP 0 187 382 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

CHEMICAL ABSTRACTS, vol. 103, no. 25, 23rd December 1985, page 272, abstract no. 208072c, Columbus, Ohio, US; T. KUDO et al.: "Molecular cloning and expression of a xylanase gene of alkalophilic Aeromonas sp. no. 212 in Escherichia coli.", & J. GEN. MICROBIOL. 1985, 131(10), 2825-30

CHEMICAL ABSTRACTS, vol. 105, no. 9, 1st September 1986, page 154, abstract no. 73432p, Columbus, Ohio, US; T. KOBAYASHI et al.: "Excretion of the penicillinase of an alkalophilic bacillus sp. through the Escherichia coli outer membrane is caused by insertional activation of the kil gene in plasmid pMB9", & J. BACTERIOL. 1986, 166(3), 728-32

## Description

Plasmid, Method for Construction of the same, Microorganism Carrying the Plasmid and Method for Cultivation of the Microorganism

This invention relates to a novel plasmid, a method for the construction of the plasmid, a novel microorganism containing the plasmid, and a method for the cultivation of the microorganism. More particularly it relates to a novel plasmid with a DNA sequence coding for the production and extracellular secretion of such high-molecular substances as xylanase or penicillinase, a novel microorganism transformed with the plasmid, and a method for the production and secretion of such high-molecular substances by culturing the microorganism.

A plasmid is a non-chromosomal gene of cyclic DNA found in a microorganism cell. Plasmids are currently being used as means for recombination of microorganism genes and are becoming more and more important in the fermentation industry.

Studies have recently been done on plasmids carrying foreign DNA having genetic information of metabolic products or specific demand for growth of microorganism, as shown in the production of amino acids or peptides. Some plasmids have been introduced into host microorganisms to obtain transformants. Methods have been proposed for producing relatively low molecular compounds such as amino acids and peptides by culturing the transformants. However, the degree of propagation of plasmids carrying genes for production of high-molecular substances depends on the nature of the host microorganisms and these plasmids have not effectively been expressed. Furthermore, no effective methods for cultivation of such transformants have been established.

It has not been possible to obtain a certain extracellular high-molecular product by culturing a microorganism transformed with a plasmid carrying a foreign DNA fragment having genetic information of extracellular secretion of high-molecular substances which are metabolic products of another microorganism. Such extracellular secretion has not been successful even using a transformant of Escherichia species, which is the most commonly used host microorganism.

USP4,411,994 of W.Gilbert et al. discloses a process for producing specific proteins in bacteria and having them excreted from the bacterial cell. This process comprises inserting the DNA representing the desired non-bacterial protein or part of a protein by recombinant techniques into a plasmid or phage gene for either a periplasmic or an extracellular protein, hereinafter called a "carrier protein", transforming a bacterial host with the recombinant gene, and culturing the transformed host to excrete the protein. The process of this patent provides a means for producing a selected protein by employing a gene for a carrier protein which has a leader sequence of hydrophobic amino acids at its amino terminus.

The cell wall of Escherichia coli, which has often been used for production of useful physiologically active substances, consists of three kinds of membranes : inner membrane, peptide glycan and outer membrane. The space between the inner and outer membrane is called the periplasmic space. The process of USP4,411,994 has succeeded in the excretion of the products within the periplasmic space but not within the culture medium through the outer membrane or outside the bacterial host cell.

The inventors of this invention previously constructed a plasmid carrying a DNA fragment prepared from chromosomal DNA of a Bacillus strain, and coding for production and extracellular secretion of penicillinase. They introduced the plasmid into Escherichia coli HB101 to obtain a transformant, Escherichia coli HB101 ($_p$EAP2), capable of producing and secreting penicillinase (Japanese Patent Unexamined Publication No. 59-162886).

Further, they succeeded in the construction of a plasmid carrying a DNA fragment prepared from chromosomal DNA of a Bacillus strain and coding for the production and extracellular secretion of xylanase. They introduced the plasmid into Escherichia coli HB101 to obtain a transformant, Escherichia coli HB101 (pCx311), having an ability to produce and secrete xylanase (Japanese Patent Application No. 58-232508).

By culturing such a strain containing a plasmid carrying a DNA fragment coding for the production and secretion of useful physiologically active substances such as enzymes, it is possible to separate and collect the active substances directly from the culture medium in a greater amount and in a more purified form because there is no need for crushing the cells of the strain and there is no saturation of the active substances within the cells.

However, useful physiologically active substances are ordinarily accumulated mainly within cells and up to now there have been known only restricted numbers of microorganisms which produce and secrete the useful physiologically active substance into media. Therefore, it is very useful from the industrial point of view to establish a method in which it is possible to create a desired strain which produces and secretes a desired useful physiologically active substance into media.

It is, therefore, an object of this invention to provide a novel plasmid which gives a microorganism transformed therewith the ability to produce and secrete a useful physiologically active substance into the fermentation medium.

Another object of this invention is to provide a method for construction of such plasmid, a novel microorganism transformed with the same and a method for culturing the microorganism to obtain a useful physiologically active substance.

This invention provides a plasmid which provides a host with the ability to produce and secrete xylanase and which comprises a DNA fragment from chromosomal DNA of a strain of the genus Bacillus coding for the production and secretion of penicillinase and a DNA fragment from chromosomal DNA of a strain of the genus Aeromonas coding for the production of xylanase.

The plasmid can be constructed by a process which comprises

(a) preparing a chromosomal DNA fragment of a strain of the genus Aeromonas coding for the production of Xylanase;

(b) cleaving a vector plasmid carrying a DNA fragment of a chromosomal DNA of a strain of the genus Bacillus with a restriction enzyme, said DNA fragment coding for the production and secretion of penicillinase; and

(c) ligating said chromosomal DNA fragment and the linearized vector plasmid with DNA ligase to form a recombinant plasmid.

This invention provides a novel microorganism which is a transformed microorganism harbouring the plasmid of the invention, having the ability to produce and secrete penicillinase and xylanase.

This invention further provides a method for the production of Xylanase, which comprises:

(a) inoculating with a microorganism according to any one of claims 9 to 11 a broth containing inorganic salts necessary for said microorganism to grow, optionally together with a carbon source; and

(b) continuing to culture the microorganism after the concentration of the microorganism cells reaches a maximum and until the production and accumulation of xylanase in the medium reach a maximum.

Brief Description of the Drawings

Fig. 1 shows a scheme for illustrating the process for construction of the plasmid, pEAP2, and the restriction enzyme cleavage map therefor.

Fig. 2 shows a scheme for illustrating the process for construction of the plasmid, pAX1, and the restriction enzyme cleavage map therefor.

Fig. 3 shows a scheme for illustrating the process for construction of the plasmid, pXP102-3, and the restriction enzyme cleavage map therefor.

Fig. 4 shows bacterial growth and production of xylanase, beta-galactosidase and beta-lactamase by Escherichia coli HB101 (pAX1).

Fig. 5 shows bacterial growth and production of xylanase by Escherichia coli HB101 (pXP102-3) of this invention.

Fig. 6 shows bacterial growth and production of penicillinase and beta-galactosidase by Escherichia coli HB101 (pXP102-3) of this invention.

The term "useful physiologically active substance" used in the specification means a high-molecular weight physiologically active compound such as an enzyme, e.g., penicillinase, xylanase, beta-galactosidase, beta-lactamase or alkaline phosphatase, or insulin, human growth hormone, interferon, antibiotic, etc.

According to this invention, there is provided a plasmid which provides a host with an ability to produce and secrete both penicillinase and xylanase. The plasmid is constructed by inserting a DNA fragment coding for the production and secretion of penicillinase (hereinafter referred to as "penicillinase DNA fragment") and a DNA fragment coding for the production of xylanase (hereinafter referred to as "xylanase DNA fragment") into a vector plasmid.

The plasmid of this invention can be constructed by inserting the xylanase DNA fragment into the cleavage site of a plasmid vector having the penicillinase DNA fragment inserted thereinto.

Vector DNA

The vector DNA which can be used in this invention is constructed by inserting the penicillinase DNA fragment into a cleavage site of a plasmid such as Col $E_1$, pMB9, pSC101, R6K, lambda phage. A typical example is the pEAP2 plasmid which is constructed by inserting the penicillinase DNA fragment prepared from a strain of the genus Bacillus into pMB9 plasmid. The pEAP2 plasmid can be constructed, for

example, as follows.

Alkalophilic Bacillus No. 170 (FERM BP-467) having the ability to produce and secrete penicillinase is cultured with shaking at 30° C for 19 hours in a broth containing 2.0 g of glycerol, 5.0 g of yeast extract, 5.0 g of polypeptone, 1.0 g of $K_2HPO_4$, 0.2 g of $MgSO_4 \cdot 7H_2O$ and 10 g of $NaHCO_3$ in one liter of water and adjusted to pH 6.0. The cells in the late logarithmic phase are collected, and a chromosomal DNA is extracted therefrom by the phenol extraction method and purified.

The chromosomal DNA is digested with the restriction enzyme Hind III at 37° C for 5, 10, 20, 30 and 60 minutes to obtain DNA fragments.

Plasmid pMB9 (Bethesda Research Laboratories, U.S.A., tetracyclin resistant ($Tet^r$)) used as a vector is cut with Hind III, then heated at 65° C for 5 minutes, and then mixed with the DNA fragments. The mixture is treated with $T_4$ phage DNA ligase and then heated at 65° C for 5 minutes.

Three-fold volume of ethanol is added to the mixture. Plasmids carrying the chromosomal DNA fragments are precipitated and collected.

The plasmids thus obtained are introduced into a host. The transformant having the desired characteristics is selected and propagated to clone the pEAP2 plasmid. As such a host, a microorganism of the genus Escherichia can be suitably used and one typical example is Escherichia coli HB101 which is a hybrid strain of Escherichia coli K-12 and Escherichia coli B. (T. Maniatis, E.F. Fritsch, J. Sambrook, Molecular Cloning, A Laboratory Manual, p.504 (1982), Genotype: $F^-$, hsd S

$$20(r_B^-, m_B^-),$$

rec A13, ara-14, proA2, lacYl, galK2, rpsL20($Sm^r$), xyl-5, mtl-1, supE44 $^-$).

Transformation with the pEAP2 plasmid will now be illustrated.

Escherichia coli HB101 is inoculated in 10 ml of LB-broth (containing 10 g of tryptone (Difco Laboratories, Detroit, Mich.), 5 g of yeast extract, 1 g of glucose and 10 g of NaCl in one liter of deionized water; pH is adjusted to 7.0) and culturing is carried out at 37° C with shaking until the late logarithmic phase. The cells are collected and suspended in an ice-cooled 0.03M $CaCl_2$ solution (final concentration) to obtain competent cells. The cell suspension and the plasmid solution are combined and kept on ice for 60 minutes. The mixture is heated to 42° C for 1 to 2 minutes to introduce the plasmid DNA into the cells. This cell suspension is inoculated in fresh LB-broth and cultured at 37° C for 3 to 5 hours with shaking. The cells are collected and washed to obtain transformants, and Escherichia coli HB101 (pEAP2) (FERM BP-468) is isolated therefrom.

Escherichia coli HB101 (pEAP2) has the same microbiological properties as the host Escherichia coli HB101 except that it is penicillin resistant and has the ability to produce and secrete penicillinase. The transformant Escherichia coli HB101 (pEAP2) is propagated. The cells are collected and the pEAP2 plasmid are isolated.

The plasmid pEAP2 is a cyclic DNA molecule of about 7.7 kb containing the penicillinase DNA fragment of about 2.0 kb prepared from the chromosomal DNA of Bacillus No. 170, the fragment being inserted into the Hind III restriction site of the plasmid pMB9.

The process for the construction of the plasmid pEAP2 and the restriction enzyme cleavage map of it are as shown in Fig. 1.

Preparation of the xylanase DNA fragment

An illustrative example of a microorganism containing the xylanase DNA fragment is a strain of the genus Aeromonas, one example of which is Aeromonas sp. No. 212 (ATCC 31085) The American Type Culture Collection Catalogue of Strains I, 14th edition 1980; (Horikoshi K, Akiba T (1982) Alkalophilic Microorganisms, P 158 Springer, Berlin Heidelberg New York). Aeromonas sp. No. 212 (ATCC 31085) is cultured in a broth (containing 0.5% ammonium sulfate, 1.5% pulp flock, 0.02% glucose, 0.1% yeast extract, 0.02% $MgSO_4 \cdot 7H_2O$ and 0.2% $K_2HPO_4$ which has been adjusted to pH9 with 7% $NaHCO_3$ solution in water) with shaking at 37° C for 10 hours. The cells in the late logarithmic phase are collected, and the chromosomal DNA is extracted therefrom by the phenol extraction method and purified.

Cloning of the chromosomal DNA is carried out as follows. The chromosomal DNA is treated with the restriction enzyme Hind III to obtain DNA fragments. A vector plasmid, pBR322 (Bethesda Research Laboratories, U.S.A., tetracyclin resistant ($Tc^r$) and ampicillin resistant ($Amp^r$)), is fully cleaved with Hind III, then heated at 65° C for 5 minutes, and then mixed with the DNA fragments. The mixture is treated with $T_4$ phage DNA ligase to insert the fragments into the Hind III restricted site of the plasmid pBR322. Ethanol is

added to the mixture. Plasmids carrying the chromosomal DNA fragments are precipitated and collected.

Escherichia coli HB101 is inoculated in 10 ml of LB broth (not containing glucose but containing 10 g of tryptone (Difco Laboratories, Detroit, Mich.), 5 g of yeast extract, 20 μg of ampicillin and 10 g of NaCl in one liter of deionized water and adjusted to pH 7.0) and cultured at 37° C with shaking until the late logarithmic phase. The cells are collected and suspended under ice-cooling in 0.03 M $CaCl_2$ solution (final concentration) in water to obtain competent cells. The cell suspension and the plasmid solution are combined and kept under ice-cooling for 60 minutes. The mixture is heated at 42° C for 1 to 2 minutes to introduce the plasmid DNA into the cells. This cell suspension is inoculated in a broth containing 100 gamma of ampicillin and 0.5% of xylan and cultured. A strain having ampicillin resistance and xylanase activity is isolated.

The strain thus obtained contains the plasmid pAX1 in which the chromosomal DNA fragment has been inserted and it has the ability to produce xylanase but not to secrete it. The strain is propagated and the cells are collected, whereafter the plasmid pAX1 is isolated therefrom. The plasmid pAX1 is cleaved with Hind III to obtain a cloned xylanase DNA fragment.

The plasmid pAX1 is a cyclic DNA molecule of about 10.4 kb containing the xylanase DNA fragment of about 6.0 kb prepared from the chromosomal DNA of Aeromonas sp. No. 212, the fragment being inserted into the Hind III restricted site of the plasmid pBR322.

The process for construction of the plasmid pAX1 and the restriction enzyme cleavage map of it are as shown in Fig. 2.

Construction of the plasmid of this invention

The plasmid of this invention can be constructed by conventional methods, e.g. the shot-gun cloning method, for inserting a DNA fragment coding for the production of a useful physiologically active substance (e.g., the xylanase DNA fragment) into a vector DNA. Construction of the plasmid of this invention will now be explained by the following example in which the plasmid pEAP2 is used as a vector plasmid and a xylanase DNA fragment obtained by cleaving the plasmid pAx1 containing the chromosomal DNA of Aeromonas sp. No. 212 with Bgl II.

The plasmid pAX1 containing the xylanase DNA fragment is cleaved with the restriction enzyme Bgl II at 37° C for 60 minutes to obtain the xylanase DNA fragment . The vector plasmid pEAP2 containing the penicillinase DNA fragment is fully cleaved with the restriction enzyme BamHI, then heated at 65° C for 5 minutes, and mixed with the xylanase DNA fragment . The mixture is treated with $T_4$ phage DNA ligase to insert the fragment into the Bam HI restriction site of the plasmid pEAP2 and then heated at 65° C for 5 minutes.

The resulting mixture of plasmids including the plasmid pXP102-3 is treated again with Bam HI and Bgl II and then with T4 phage DNA ligase in order to increase the concentration of the plasmid pXP102-3.

Escherichia coli HB101 is inoculated in 10 ml of LB broth (not containing glucose but containing 10 g of tryptone (Difco), 5 g of yeast extract, 20 μg of ampicillin and 10 g of NaCl and adjusted to pH 7.0) and cultured at 37° C with shaking until the late logarithmic phase. The cells are collected and suspended under ice-cooling in 0.03 M $CaCl_2$ solution (final concentration) in water to obtain competent cells. The cell suspension and the plasmid solution are combined and kept under ice-cooling for 60 minutes. The mixture is heated at 42° C for 1 to 2 minutes to introduce the plasmid DNA into the cells. This cell suspension is inoculated in a broth containing 20 μg of ampicillin and 0.5% of xylan and cultured. A strain having ampicillin resistance and xylanase activity is isolated.

The transformant Escherichia coli HB101 (pXP102-3) contains the plasmid pXP102-3 in which the chromosomal DNA has been inserted and it has the ability to produce and secrete both penicillinase and xylanase.

The strain Escherichia coli HB101 (pXP102-3) is propagated and the cells are collected, whereafter the plasmid pXP102-3 is isolated therefrom.

The plasmid pXP102-3 is a cyclic DNA molecule of 11.7 kb containing the xylanase DNA fragment prepared from the chromosomal DNA of Aeromonas sp. No. 212, the fragment being inserted into the BamHI restriction site of the plasmid pEAP2.

The process for construction of the plasmid pXP102-3 and the restriction enzyme cleavage map of it are as shown in Fig. 3.

Escherichia coli HB101 (pXP102-3) has the same microbiological properties as the host, Escherichia coli HB101 except that it has penicillin resistance and the ability to produce and secrete both penicillinase and xylanase.

Thus, Escherichia coli HB101 (pXP102-3) can be cultured under the same cultivation conditions as the parent strain, Escherichia coli HB101, to produce and secrete both penicillinase and xylanase.

Cultivation of the microorganism

For culturing the transformant of this invention, there can be used any culture media suitable for the production of specific substances for which a specific genetic information codes and suitable for the growth of the microorganisms of the genus Escherichia. In the process of the present invention, it is possible to culture the microorganism in a culture medium containing inorganic salts necessary for the microorganism to grow together with or without a carbon source, and to continue culturing the microorganism after the concentration of the microorganism cells reaches a maximum, and until the production and accumulation of the physiologically active substances in the media reach a maximum. It is preferred to use a medium not containing a carbon source as described below for accelerating the secretion of the physiologically active substance.

Examples of inorganic salts which can be used in the present invention include sodium and potassium salts such as sodium chloride, sodium sulfate, potassium chloride and the like, among which sodium chloride is preferred. Examples of carbon sources include glucose, sucrose, lactose, maltose, glycerol, bran, xylan and the like. The media which can be used in this invention may contain nitrogen sources as ammonia water, ammonium salts and the like, inorganic ions and optionally such nutrients as amino acids, vitamins and the like Typical examples of the media which can be used in the present invention are those which use, as a basic medium, LB-broth (containing tryptone, yeast extract and NaCl), BPB-broth (Difco Laboratories; containing polypeptone, yeast extract and $K_2HPO_4$), nutrient broth (Difco 0001), tryptone-NaCl broth or the like.

Since almost all products remain in the intracellular space in a medium containing no inorganic salt, it is necessary to use a medium containing an inorganic salt so that most products will be secreted out of the cells into the culture medium. The amount of inorganic salt used is in the range of 0.1 - 3.0% by weight on the basis of the culture medium.

Although culture conditions such as pH, temperature, oxygen supply or the like can be adjusted for the optimum growth of the microorganism of the genus Escherichia, it is necessary in the present invention to continue culturing the microorganism after the concentration of the microorganism cells reaches a maximum, that is to say, after the late logarithmic phase and until the production and accumulation of the high-molecular substances in the medium reach a maximum.

After the inoculation of the microorganism of the genus Escherichia in the medium, the cell concentration reaches a maximum within 5 to 20 hours and the production and accumulation of the high-molecular substances reach a maximum within 12 to 48 hours. Although the pH of the culture medium is not critical, it is preferred to be in the range of pH5 - pH8, with a pH of 7 being especially preferred. Thus, without further addition of inorganic salt necessary for the microorganism to grow, carbon sources and the like during cultivation, the microorganism produces and secretes large amounts of physiologically active substances which can readily be collected.

According to the culture method of the present invention, in addition to enzyme proteins, such fermentation products as antibiotics or polysaccharides can be produced in a significant amount in the media. This method can be applied to the cultivation of any microorganisms transformed with a plasmid carrying a foreign DNA coding for biologically active substances such as hormone peptides (e.g. insulin) or interferon and the DNA coding for production and secretion of biologically active substances, which enables mass production of biologically active substances such as insulin, interferon and the like.

As explained above, the present invention contributes to the industrial production of useful biologically active substances.

Examples of microorganisms which may be used in this invention include (i) Bacillus sp. No. 170, (ii) Escherichia coli HB101 (pEAP2), (iii) Aeromonas sp. No. 212, (iv) Escherichia coli HB101 (pAX1) and (v) Escherichia coli HB101 (pXP102-3). The microorganisms (i), (ii), (iv) and (v) were deposited with the Fermentation Research Institute, Agency of Industrial Science and Technology, Ministry of International Trade and Industry, Japan, International Depository Authority (hereinafter referred to as "FERM") under the following accession numbers, respectively, FERM BP-467, FERM BP-468, FERM BP-950 and FERM BP-951 and are on deposit with FERM in an unrestricted deposit permitting the full access to the culture, and the microorganism (iii) was deposited with the American Type Culture Collection under the accession number of ATCC 31085.

The depository and accession numbers of the above mentioned microorganisms are shown below:

| Microorganisms | Depository | Accession No. |
|---|---|---|
| (i) Bacillus sp. No. 170 | FERM | FERM BP-467 |
| (ii) Escherichia coli HB101 (pEAP2) | FERM | FERM BP-468 |
| (iii) Aeromonas sp. No. 212 | ATCC | ATCC 31085 |
| (iv) Escherichia coli HB101 (pAX1) | FERM | FERM BP-950 |
| (v) Escherichia coli HB101 (pXP102-3) | FERM | FERM BP-951 |

This invention provides a plasmid which can be constructed by inserting a chromosomal DNA fragment coding for production but not secretion of the useful physiologically active substance xylanase into a restriction enzyme cleavage site of a vector plasmid which provides a host with the ability to produce and secrete penicillinase. The plasmid thus constructed provides a host with the ability to produce and secrete the useful physiologically active substance xylanase in addition to penicillinase. It is possible, to construct a new plasmid which provides a host such as Escherichia coli with the ability of production and secretion of useful physiologically active substances such as insulin, human growth hormone, interferon, enzyme, and the like and to create a useful microorganism having the ability to produce and secrete such useful substance.

We will now explain, with reference to the Examples, methods for the construction of the plasmids of the present invention, methods for the preparation of the transformant, Escherichia coli HB101 (pXP102-3), and the production and secretion of penicillinase, xylanase and so on by the transformant.

Example 1 Construction of the plasmid pEAP2

Alkalophilic Bacillus sp. No. 170 (FERM BP-467) having the ability to produce and secrete penicillinase was cultured at 30°C with shaking for 19 hours in a broth (containing 2.0 g of glycerol, 5.0 g of yeast extract, 5.0 g of polypeptone, 1.0 g of $K_2HPO_4$, 0.2 9 of $MgSO_4 \cdot 7H_2O$, 10 g of $NaHCO_3$ in one liter of deionized water; pH 9.0). The cells in the late logarithmic phase were collected, and chromosomal DNA was extracted therefrom by the phenol extraction method and purified to obtain 5 mg of the chromosomal DNA.

The chromosomal DNA (10 micrograms) obtained was digested with the restriction enzyme Hind III at 37°C for 5, 10, 20, 30 and 60 minutes to obtain DNA fragments.

Plasmid pMB9 (Bethesda Research Laboratories, U.S.A., tetracyclin resistant ($Tet^r$)) used as a vector was cleaved with Hind III, then heated at 65°C for 5 minutes, and then mixed with the DNA fragments. The mixture was treated with $T_4$ phage DNA ligase at 10°C for 24 hours and then heated at 65°C for 5 minutes.

Two-fold volume of ethanol was added to the mixture. Plasmids carrying the chromosomal DNA fragments were precipitated and collected.

Escherichia coli HB101, which is a hybrid strain of Escherichia coli K-12 and Escherichia coli B, was inoculated in 10 ml of LB-broth (containing 10 g of tryptone (Difco Laboratories, Detroit, Mich.), 5 g of yeast extract, 1 g of glucose and 10 g of NaCl in one liter of deionized water; pH was adjusted to 7.0) and cultured at 37°C with shaking until the late logarithmic phase. The cells were collected and suspended under ice-cooling in a 0.03M $CaCl_2$ solution (final concentration) to obtain competent calls. The cell suspension and the plasmid solution ware combined and kept under ice-cooling for 60 minutes. The mixture was heated at 42°C for 1 to 2 minutes to introduce the plasmid DNA into the cells. This cell suspension was inoculated in fresh LB-broth and cultured at 37°C for 3 to 5 hours with shaking. The cells were collected and washed to obtain transformants, from which Escherichia coli HB101 (pEAP2) (FERM BP-468) was isolated. The cells were treated as follows to obtain purified plasmid pEAP2.

Cell pellets

10 ml, 20% Sucrose, 50 mM Tris, 1 mM EDTA pH8. Suspended, Keep on ice.

50 ml polypropylene centrifuge tube.

↓ 2 ml, 0.25 M EDTA.
1 ml, Lysozyme (5 mg/ml of 0.025 M Tris, pH8.)
0.1 ml, R Nases (10 mg/ml)

Cell lysis. Mix gently. Stand 15 to 30 min. on ice.

↓ 5 ml, 3x Triton. Mix gently. Stand 15 to 45 min. on ice.

Centrifugation. 17,000 rpm, 40 min. at 4°C.

↓

Supernatant soln. in plastic cylinder.

↓

250 ml glass bottle.

↓ 2/3 vol DD $H_2O$
2/3 vol Cold saturated phenol. Mix gently

Centrifugation. 6,500 rpm, 15 min. at 4°C.

↓

Upper phase.

↓ Equal vol. of phenol; Chloroform.

Centrifugation. 6,500 rpm, 15 min. at 4°C.

↓

Upper phase in 250 ml bottle.

↓ 1/25 vol 5 M NaCl.
2 vol EtOH at -20°C.
Over night at -20°C.

Centrifugation. 6,500 rpm, 60 min. at -20°C.

↓

DNA pellet. Dry excess liquid.

5 ml A-50 buffer, resuspended.
1 ml sterile 80% glycerol, mix gently.

↓
A-50 column. (2x35 cm, 1 fraction = 4 ml.)
↓
DNA fraction. ($A_{260}$ peak.)
↓ 2 vol EtOH at -20°C. Over night at -20°C.
Centrifugation. 6,500 rpm, 60 min. at -20°C.
|
DNA pellet.
↓ 2.1 ml TEN buffer in 5 ml nitrocellulose tube.
2.2 g CsCl, mix.
(In dark.)
150 μl PdI (2 mg/ml). Mix well.
2 ml mineral oil on top of tube.
CsCl gradient centrifugation. 36,000 rpm, 40 hr at 20°C.
↓ Visible with UV.
Upper band; chromosomal & nicked DNA.
Lower band; covalently closed p-DNA.
Collect lower band DNA drop wise
↓
Dowex 50W-X8 column. UV check.
↓ (In light.)
Dialysis against 2 to 4 liters of 10 mM Tris, 1 mM EDTA PH8.
↓ Over night at 4°C.
In 30 ml cortex centrifuge tube.
↓ 1/25 vol 5 M NaCl.
2 vol EtOH at -20°C.
Over night at -20°C.
Centrifugation. 6,500 rpm, 60 min. at -20°C.
↓
DNA precipitates.
↓ 1-2 ml TEN buffer.
Purified plasmid DNA (1 mg/l-broth). Stored at -20 to 70°C.

Example 2 Preparation of the xylanase DNA fragment

Aeromonas sp. No. 212 (ATCC 31085) having the ability to produce xylanase was cultured in a broth (containing 0.5% ammonium sulfate, 1.5% pulp flock, 0.02% glucose, 0.1% yeast extract, 0.02%

$MgSO_4 \cdot 7H_2O$ and 0.2% $K_2HPO_4$ and adjusted to pH9 with a 7% $NaHCO_3$ solution in water) at 37° C for 10 hours with shaking until the late logarithmic phase. The cells were collected, and chromosomal DNA was extracted therefrom by the phenol extraction method and purified to obtain 3 mg of the chromosomal DNA.

The chromosomal DNA (10 micrograms) was digested with Hind III at 37° C for 5, 10, 20, 30 and 60 minutes to obtain DNA fragments.

Plasmid pBR 322 (Bethesda Research Laboratories, U.S.A., tetracyclin resistant ($Tet^r$) and ampicillin resistant ($Amp^r$)) used as a vector was fully cleaved with Hind III, then heated at 65° C for 5 minutes, and then mixed with the DNA fragments. The mixture was treated with $T_4$ ligase at 10° C for 24 hours and then heated at 65° C for 5 minutes. Two-fold volume of ethanol was added to the mixture. Plasmids carrying the chromosomal DNA fragments were precipitated and collected.

Escherichia coli HB101 was inoculated in 10 ml of LB broth (containing 10 g of tryptone (Difco Laboratories), 5 g of yeast extract, 20 μg of ampicillin and 10 g of NaCl but not containing glucose; pH 7.0) and cultured at 37° C with shaking until the late logarithmic phase. The cells were collected and suspended under ice-cooling in a 0.03 M $CaCl_2$ solution in water (final concentration) to obtain competent cells. The cell suspension and the plasmid solution were combined and kept under ice-cooling for 60 minutes. The mixture was heated at 42° C for 1 to 2 minutes to introduce the plasmid DNA into the cells. The cell suspension was inoculated in a broth containing 100 μg of ampicillin and 0.5% of xylan and cultured. Four strains having xylanase activity were isolated from among 3,000 strains. The four strains were cultured in LB broth, from which plasmids were isolated and purified in the same manner as in Example 1. The analysis of the sequence structure of the plasmids showed that the same DNA fragment had been inserted in the same direction. It was confirmed by Southern's method that the DNA fragment was one derived from Aeromonas sp. No. 212.

Example 3 Construction of pXP102-3

The plasmid pAX1 (5 micrograms) carrying the xylanase DNA fragment was cleaved with the restriction enzyme Bgl II (15 units) at 37° C for 60 minutes to obtain DNA fragments.

The plasmid pEAP2 (5 micrograms) carrying the penicillinase DNA fragment was fully cleaved with Hind III at 37° C for 60 minutes, then treated at 65° C for 5 minutes and then mixed with the DNA fragments obtained from the plasmid pAX1. The mixture was treated with $T_4$ phage DNA ligase and then heated at 65° C for 5 minutes.

To the mixture, there were added Bam HI (15 units) and Bgl II (15 units) and then the mixture was treated at 37° C for 60 minutes to selectively cleave pEAP2 and pAX1 and subsequently treated with $T_4$ phage DNA ligase so that the concentration of the plasmid pXP102-3 in the mixture was increased.

Escherichia coli HB101 was inoculated in LB broth not containing glucose and cultured at 37° C with shaking until the late logarithmic phase. The cells were collected and suspended under ice-cooling in an aqueous 0.03 M $CaCl_2$ solution (final concentration) to obtain competent cells. The cell suspension and the plasmid solution were combined and kept under ice-cooling for 60 minutes. The mixture was then heated at 42° C for 1 to 2 minutes to introduce the plasmid into the cells. The cell suspension was inoculated in LB broth not containing glucose and cultured. A strain having ampicillin resistance and xylanase activity was isolated. The transformant Escherichia coli HB101 (pXP102-3) (FERM BP-951) was obtained, which was then treated in the same manner as in Example 1 to obtain the plasmid pXP102-3.

Example 4

Escherichia coli HB101 (pAX1) (FERM BP-950) and Escherichia coli HB101 (pXP102-3) (FERM BP-951) obtained in Examples 2 and 3, respectively were cultured at 37° C with shaking in LB broth not containing glucose (containing 10 g of tryptone (Difco), 5 g of yeast extract, 20 μg of ampicillin and 10 g of NaCl in one liter of water and adjusted to pH 7.0). The cell growth (cell weight) was monitored by measuring the optical density at 660 nm. Penicillinase activity was determined by a modified Sargent's method (Sawai et al: Antimicrob. Agents Chemother. 13, 910 (1978)), wherein the amount of penicillinase hydrolysing one micromole of benzyl penicillin per minute at 30° C was taken as one unit. Xylanase activity was determined as follows: To 0.05 ml of enzyme solution, there were added 0.1 ml of xylan solution (produced by SEI-KAGAKU KOGYO, Japan) and 0.1 ml of 0.2M Tris malate buffer (pH 8.0) and the reaction was allowed to proceed at 40° C for 10 minutes. DNS (3.5-dinitrosalicylic acid) (1 ml) was added and reacted at 100° C for 5 minutes. Water (4 ml) was added and absorbance at 510 nm was measured. The amount of enzyme showing a reducing power producing 1 mg of xylose per minute was taken as one unit of xylanase.

Beta-galactosidase activity was determined as follows: 100 microliters of enzyme solution and 900

microliters of Z buffer (containing 4.3 g of $NaHPO_4 \cdot 12H_2O$, 1.25 g of $NaH_2PO_4$, 0.15 g of KCl and 0.04 g of $MgSO_4$ in 200 ml of distilled water) were reacted at 30°C for 5 minutes. Arthonitropyranosyl galactoside (0.2 ml) (4 mg/ml in 0.1 M phosphate buffer (pk 7)) was added and reacted at 30°C for 10 minutes. A 2% $Na_2CO_3$ solution in water (1.8 ml) was added and absorbance at 420 nm was measured. The enzyme activity was expressed as the increase in the absorbance per ml of enzyme solution per minute.

Beta-lactamase activity was determined in the same way as for penicillinase.

Fig. 4 shows the growth and production of xylanase, beta-galactosidase and beta-lactamase by Escherichia coli HB101 (pAX1). Xylanase was accumulated mainly inside the cell and in the periplasmic space but slightly outside the cell. Beta-galactosidase was accumulated mainly inside the cell but slightly in the periplasmic space and outside the cell. Beta-lactamase was accumulated first in the periplasmic space but it was not found therein after 20 hours.

Fig. 5 shows production of xylanase by Escherichia coli HB101 (pXP102-3). Almost all xylanase produced was secreted outside the cell within 16 to 20 hours. Xylanase was found in a small amount in the periplasmic space but not inside the cell.

The transformant, Escherichia coli HB101 (pXP102-3), containing the plasmid pXP102-3 which was constructed by inserting the xylanase DNA fragment obtained from Escherichia coli HB101 (pAX1) which produces xylanase but does not secrete it into media, into the plasmid pEAP2 of Escherichia coli HB101 (pEAP2) which produces penicillinase and secretes it into media, produces and secretes into media not only penicillinase but also xylanase.

Fig. 6 shows production of penicillinase and beta-galactosidase by Escherichia coli HB101 (pXP102-3). Almost all penicillinase produced was secreted and accumulated outside the cell. In contrast, almost all beta-galactosidase was accumulated inside the cell and was not secreted outside the cell.

## Claims

1. A plasmid which provides a host with the ability to produce and secrete xylanase and which comprises a DNA fragment from chromosomal DNA of a strain of the genus Bacillus coding for the production and secretion of penicillinase and a DNA fragment from chromosomal DNA of a strain of the genus Aeromonas coding for the production of xylanase.

2. The plasmid of claim 1, wherein said strain of the genus Bacillus is Bacillus No. 170 (FERM BP-467).

3. The plasmid of claim 1 or 2, wherein said strain of the genus Aeromonas is Aeromonas sp. No. 212 (ATCC 31085).

4. The plasmid of claim 1 or 2, which is the plasmid pXP102-3 (FERM BP-951).

5. The plasmid of any of claims 1 to 4, wherein said host is a strain of the genus Escherichia.

6. The plasmid of claim 5, wherein said host is Escherichia coli.

7. A method for the construction of the plasmid according to any one of claims 1 to 6, which comprises:
   (a) preparing a chromosomal DNA fragment of a strain of the genus Aeromonas coding for the production of xylanase;
   (b) cleaving a vector plasmid carrying a DNA fragment of a chromosomal DNA of a strain of the genus Bacillus with a restriction enzyme, said DNA fragment coding for the production and secretion of penicillinase; and
   (c) ligating said chromosomal DNA fragment and the linearized vector plasmid with DNA ligase to form a recombinant plasmid.

8. The method of claim 7, wherein said vector plasmid is the plasmid pEAP2 having the restriction map given in Fig. 1.

9. A transformed microorganism harbouring a plasmid according to any one of claims 1 to 6.

10. The transformant according to claim 9 which is a microorganism of the genus Escherichia.

11. The transformant according to claim 9 which is Escherichia coli HB101 (pXP102-3) (FERM BP-951)

having the ability to produce and secrete penicillinase and xylanase.

12. A method for the production of xylanase, which comprises:
(a) inoculating with a microorganism according to any one of claims 9 to 11 a broth containing inorganic salts necessary for said microorganism to grow, optionally together with a carbon source; and
(b) continuing to culture the microorganism after the concentration of the microorganism cells reaches a maximum and until the production and accumulation of xylanase in the medium reach a maximum.

13. The method of claim 12, wherein the inorganic salt is a sodium salt or a potassium salt, and the carbon source is bran or xylan.

14. The method of claim 12 or 13, wherein the inorganic salt is used in an amount of 0.1 to 3.0% by weight on the basis of the weight of the medium.

15. The method of any one of claims 12 to 14, wherein said microorganism is cultured for 12 to 48 hours.

**Revendications**

1. Plasmide qui fournit à un hôte la possibilité de produire et de secréter de la xylanase et qui comprend un fragment d'ADN provenant de l'ADN chromosomique d'une souche du genre Bacillus codant pour la production et la sécrétion de pénicillinase, et un fragment d'ADN provenant de l'ADN chromosomique d'une souche du genre Aeromonas codant pour la production de xylanase.

2. Plasmide de la revendication 1, dans lequel ladite souche du genre Bacillus est le Bacillus n° 170 (FERM BP-467).

3. Plasmide de la revendication 1 ou 2, dans lequel ladite souche du genre Aeromonas est Aeromonas sp. n° 212 (ATCC 31085).

4. Plasmide de la revendication 1 ou 2, qui est le plasmide pXP102-3 (FERM BP-951).

5. Plasmide de l'une quelconque des revendications 1 à 4, dans lequel ledit hôte est une souche du genre Escherichia.

6. Plasmide de la revendication 5, dans lequel ledit hôte est Escherichia coli.

7. Méthode de préparation du plasmide selon l'une quelconque des revendications 1 à 6, comprenant:
a) la préparation d'un fragment d'ADN chromosomique d'une souche du genre Aeromonas codant pour la production de xylanase;
b) le clivage d'un plasmide vecteur portant un fragment d'ADN chromosomique d'une souche du genre Bacillus avec une enzyme de restriction, ledit fragment d'ADN codant pour la production et la sécrétion de pénicillinase ; et
c) la ligation dudit fragment d'ADN chromosomique et du plasmide vecteur linéarisé avec l'ADN ligase pour former un plasmide recombinant.

8. Méthode de la revendication 7, dans laquelle ledit plasmide vecteur est le plasmide pEAP2 dont une carte de restriction est donnée dans la fig. 1.

9. Micro-organisme transformé, comportant un plasmide selon l'une quelconque des revendications 1 à 6.

10. Transformant selon la revendication 9, qui est un micro-organisme du genre Escherichia.

11. Transformant selon la revendication 9, qui est Escherichia coli HB101 (pXP102-3) (FERM BP-951) ayant la capacité de produire et de sécréter de la pénicillinase et de la xylanase.

12. Méthode de production de la xylanase, comprenant:

a) l'ensemencement avec un micro-organisme selon l'une quelconque des revendications 9 à 11 d'un bouillon de culture contenant des sels inorganiques nécessaires à la croissance dudit micro-organisme, et facultativement une source de carbone ; et
b) la poursuite de la culture du micro-organisme après que la concentration des cellules de micro-organismes atteint un maximum et jusqu'à ce que la production et l'accumulation de xylanase dans le milieu atteigne un maximum.

**13.** Méthode de la revendication 12, dans laquelle le sel inorganique est un sel de sodium ou un sel de potassium, et la source de carbone est constituée de son ou de xylane.

**14.** Méthode de la revendication 12 ou 13, dans laquelle le sel inorganique est utilisé dans une proportion de 0,1 à 3,0 % en poids sur la base du poids du milieu.

**15.** Méthode de l'une quelconque des revendications 12 à 14, dans laquelle ledit micro-organisme est cultivé pendant 12 à 48 heures.

**Patentansprüche**

**1.** Plasmid, das einen Wirt mit der Fähigkeit zur Bildung und Sekretion von xylanase ausstattet und ein DNA-Fragment der chromosomalen DNA eines Stammes der Gattung Bacillus, das für die Herstellung und Sekretion von Penicillinase codiert und ein DNA-Fragment der chromosomalen DNA eines Stammes der Gattung Aeromonas enthält, das für die Herstellung von Xylanase codiert.

**2.** Plasmid nach Anspruch 1, wobei der Stamm der Gattung Bacillus Bacillus Nr. 170 (FERM BP-467) ist.

**3.** Plasmid nach Anspruch 1 oder 2, wobei der Stamm der Gattung Aeromonas Aeromonas sp. Nr. 212 (ATCC 31085) ist.

**4.** Plasmid nach Anspruch 1 oder 2, das das Plasmid pXP102-3 (FERM BP-951) ist.

**5.** Plasmid nach einem der Ansprüche 1 bis 4, wobei der Wirt ein Stamm der Gattung Escherichia ist.

**6.** Plasmid nach Anspruch 5, wobei der Wirt Escherichia coli ist.

**7.** Verfahren zur Herstellung des Plasmids nach einem der Ansprüche 1 bis 6, bei dem man:
(a) ein chromosomales DNA-Fragment eines Stammes der Gattung Aeromonas herstellt, das für die Herstellung von Xylanase codiert;
(b) ein Vektor-Plasmid, das ein DNA-Fragment der chromosomalen DNA eines Stammes der Gattung Bacillus trägt, mit einem Restriktionsenzym spaltet, wobei das DNA-Fragment für die Herstellung und Sekretion von Penicillinase codiert; und
(c) das chromosomale DNA-Fragment und das linearisierte Vektor-Plasmid mit DNA-Ligase zu einem rekombinanten Plasmid ligiert.

**8.** Verfahren nach Anspruch 7, wobei das Vektor-Plasmid das Plasmid pEAP2 mit der in Figur 1 angegebenen Restriktionskarte ist.

**9.** Transformierter Mikroorganismus, der ein Plasmid nach einem der Ansprüche 1 bis 6 enthält.

**10.** Transformante nach Anspruch 9, die ein Mikroorganismus der Gattung Escherichia ist.

**11.** Transformante nach Anspruch 9, die Escherichia coli HB101 (pXP102-3) (FERM BP-951) ist, mit der Fähigkeit zur Herstellung und Sekretion von Penicillinase und Xylanase.

**12.** Verfahren zur Herstellung von Xylanase, bei dem man:
(a) ein Medium mit einem Mikroorganismus nach einem der Ansprüche 9 bis 11 beimpft, das für das Wachstum des Mikroorganismus erforderliche anorganische Salze gegebenenfalls in Kombination mit einer Kohlenstoffquelle enthält; und
(b) den Mikroorganismus, nachdem die Konzentration der Mikroorganismuszellen ein Maximum

erreicht hat, weiterzüchtet, bis die Herstellung und Ansammlung der Xylanase im Medium ein Maximum erreicht.

**13.** Verfahren nach Anspruch 12, wobei das anorganische Salz ein Natrium- oder Kaliumsalz und die Kohlenstoffquelle Kleie oder Xylan ist.

**14.** Verfahren nach Anspruch 12 oder 13, wobei das anorganische Salz in einer Menge von 0,1 bis 3,0 Gew.-% bezogen auf das Gewicht des Mediums verwendet wird.

**15.** Verfahren nach einem der Ansprüche 12 bis 14, wobei der Mikroorganismus 12 bis 48 Stunden gezüchtet wird.

# FIG.1

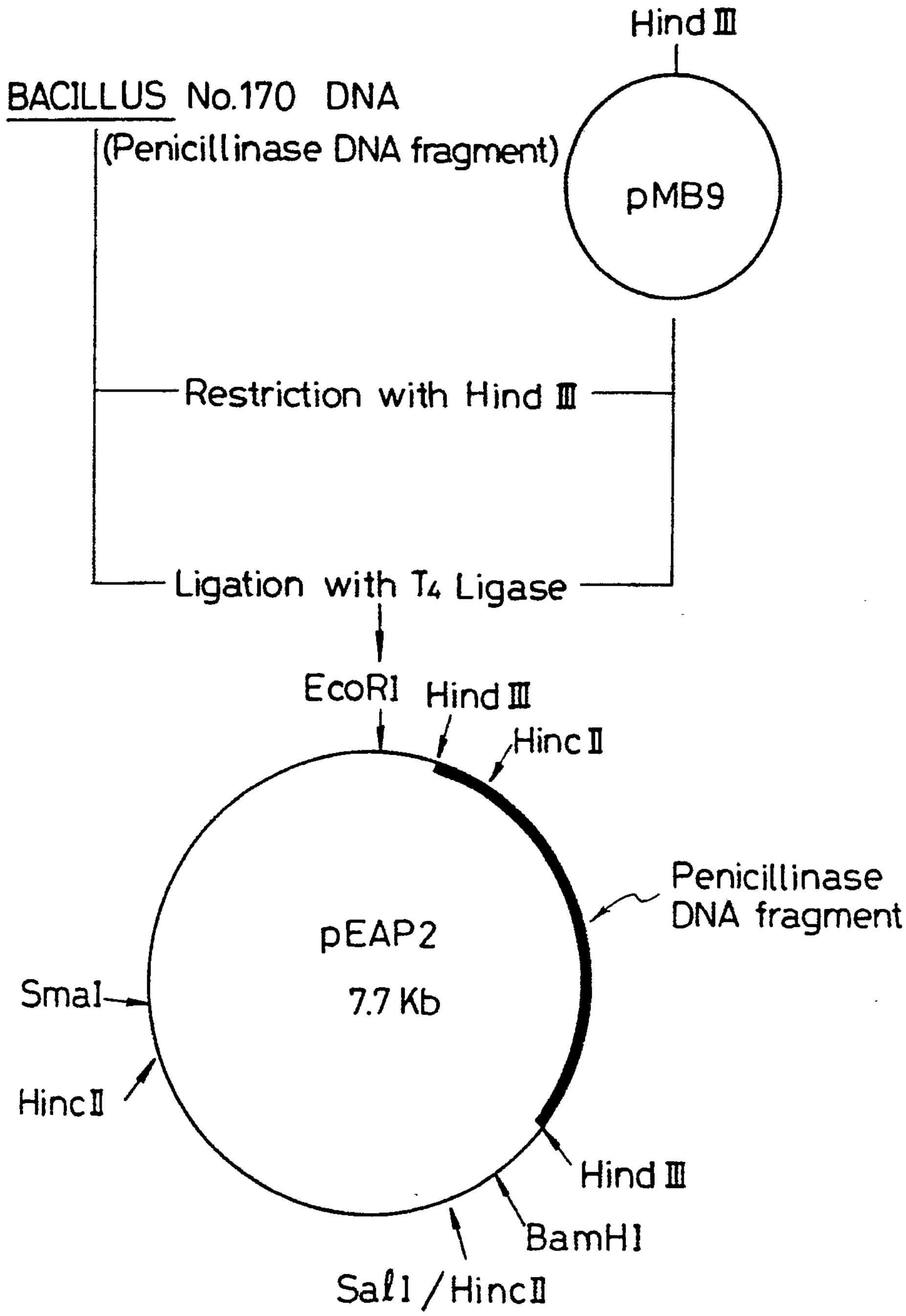
Hind III
BACILLUS No.170 DNA
(Penicillinase DNA fragment)
pMB9
Restriction with Hind III
Ligation with T4 Ligase
EcoR1
Hind III
Hinc II
Penicillinase
DNA fragment
pEAP2
7.7 Kb
Sma1
Hinc II
Hind III
BamH1
Sal1 / Hinc II

# FIG.2

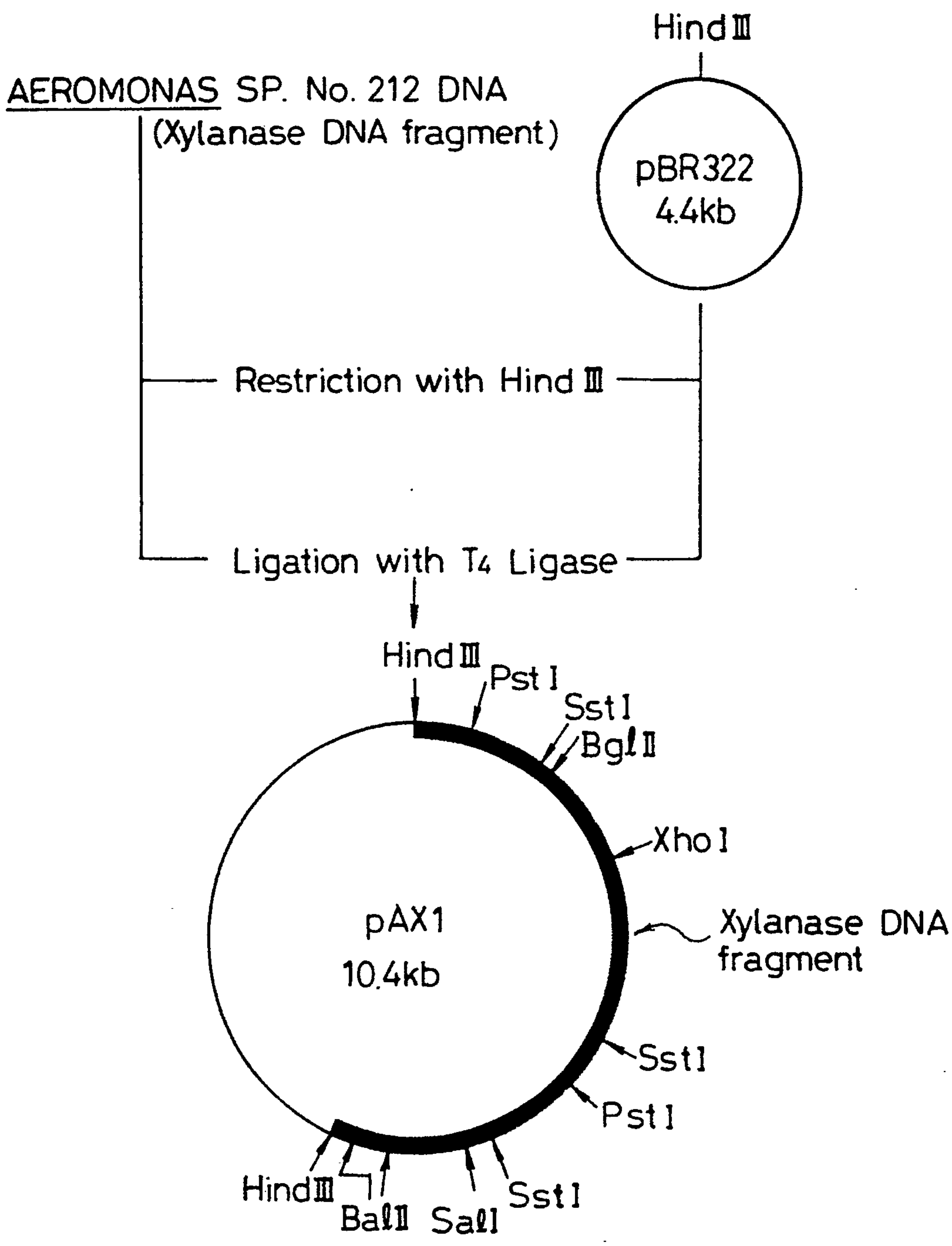
Hind III
AEROMONAS SP. No. 212 DNA
(Xylanase DNA fragment)
pBR322
4.4kb
Restriction with Hind III
Ligation with T4 Ligase
Hind III
Pst I
Sst I
Bgl II
Xho I
pAX1
10.4kb
Xylanase DNA fragment
Sst I
Pst I
Hind III
Bal II
Sal I
Sst I

# FIG.3

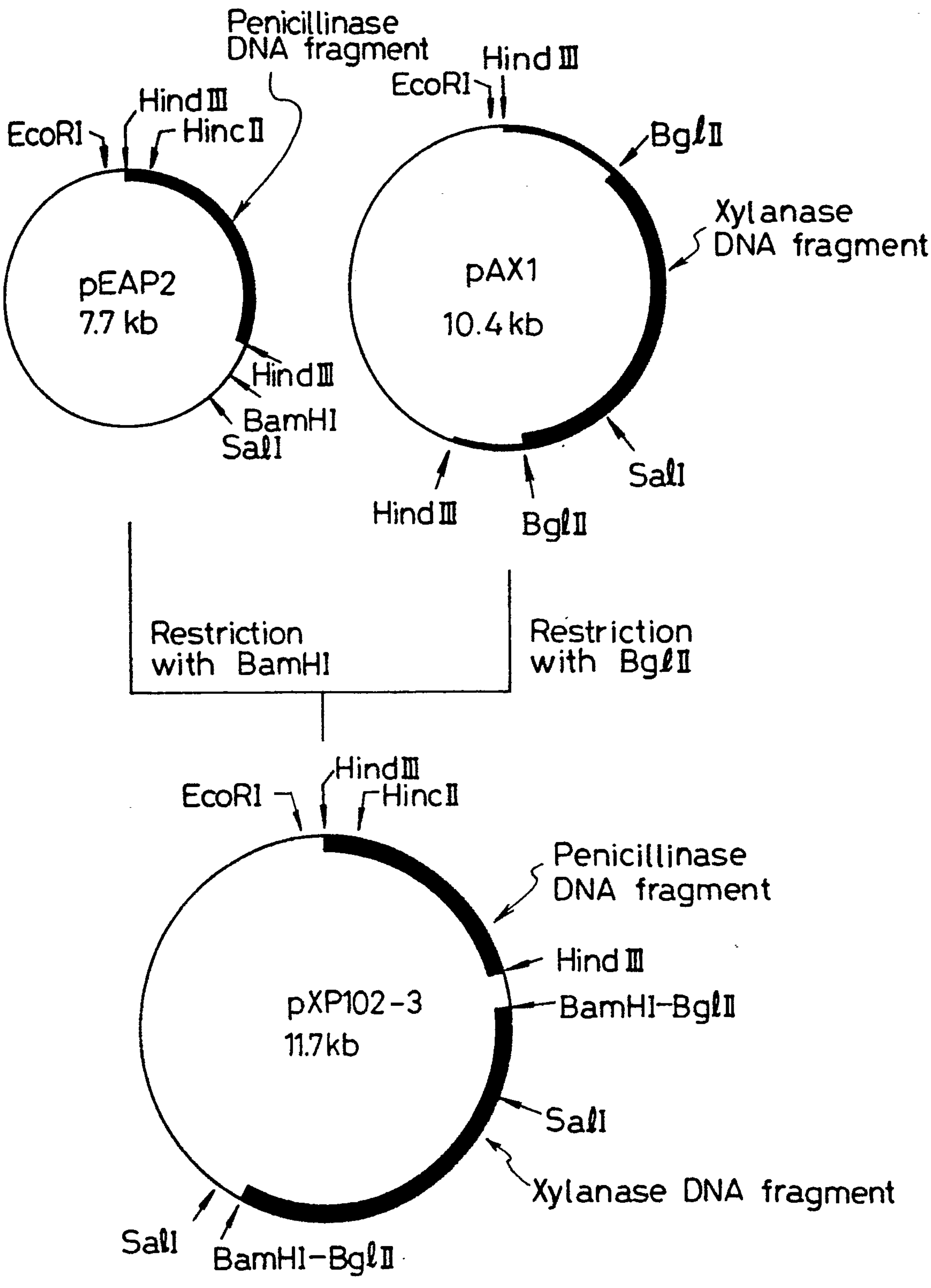
Penicillinase DNA fragment
EcoRI
HindIII
HincII
pEAP2
7.7 kb
HindIII
BamHI
SalI
EcoRI
HindIII
BglII
Xylanase DNA fragment
pAX1
10.4 kb
SalI
HindIII
BglII
Restriction with BamHI
Restriction with BglII
EcoRI
HindIII
HincII
Penicillinase DNA fragment
HindIII
BamHI–BglII
pXP102-3
11.7kb
SalI
Xylanase DNA fragment
SalI
BamHI–BglII

FIG.4

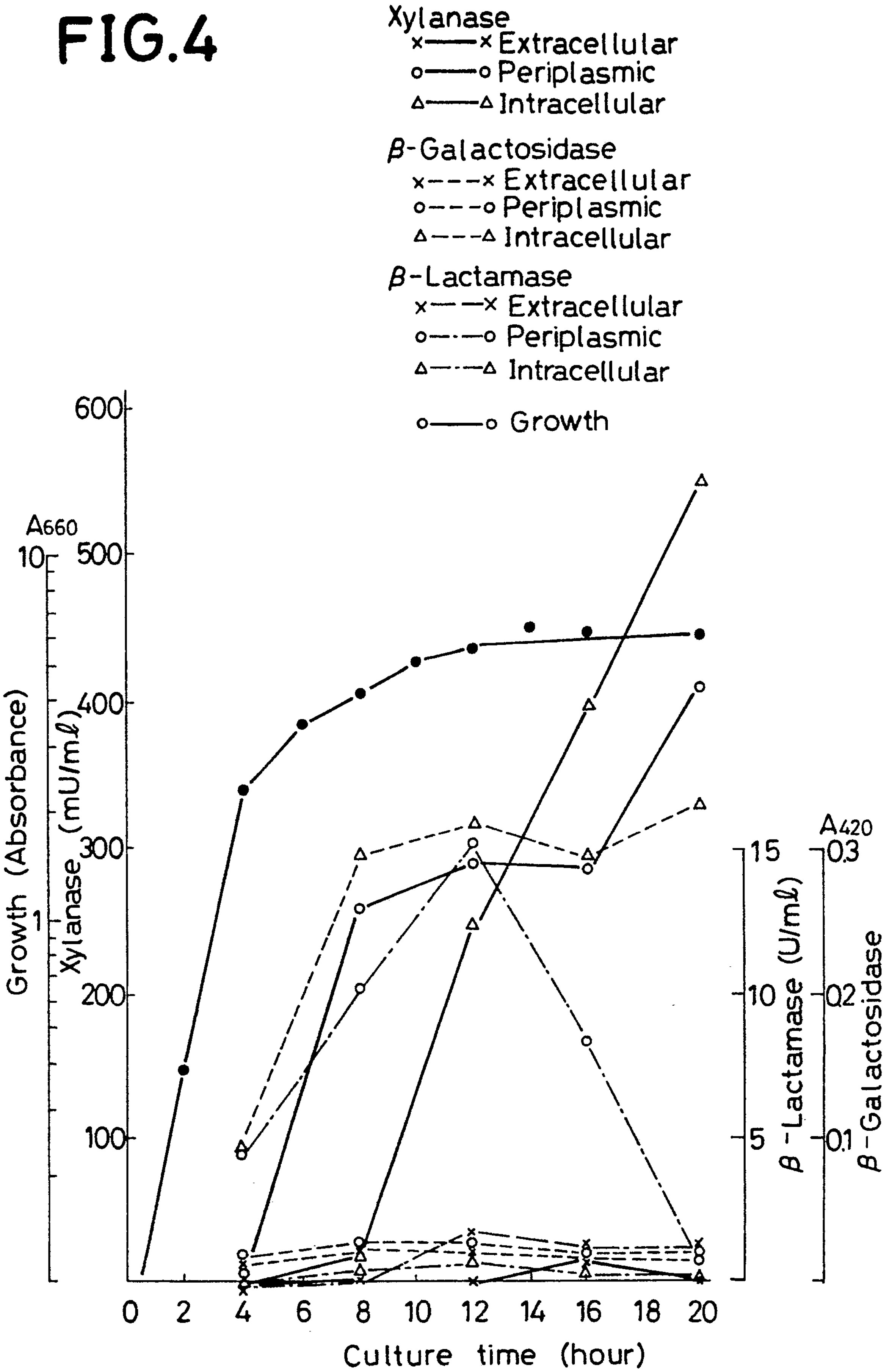
Xylanase
Extracellular
Periplasmic
Intracellular
β-Galactosidase
Extracellular
Periplasmic
Intracellular
β-Lactamase
Extracellular
Periplasmic
Intracellular
Growth
A660
10
1
600
500
400
300
200
100
Growth (Absorbance)
Xylanase (mU/mℓ)
A420
15
10
5
0.3
0.2
0.1
β-Lactamase (U/mℓ)
β-Galactosidase
0 2 4 6 8 10 12 14 16 18 20
Culture time (hour)

FIG.5

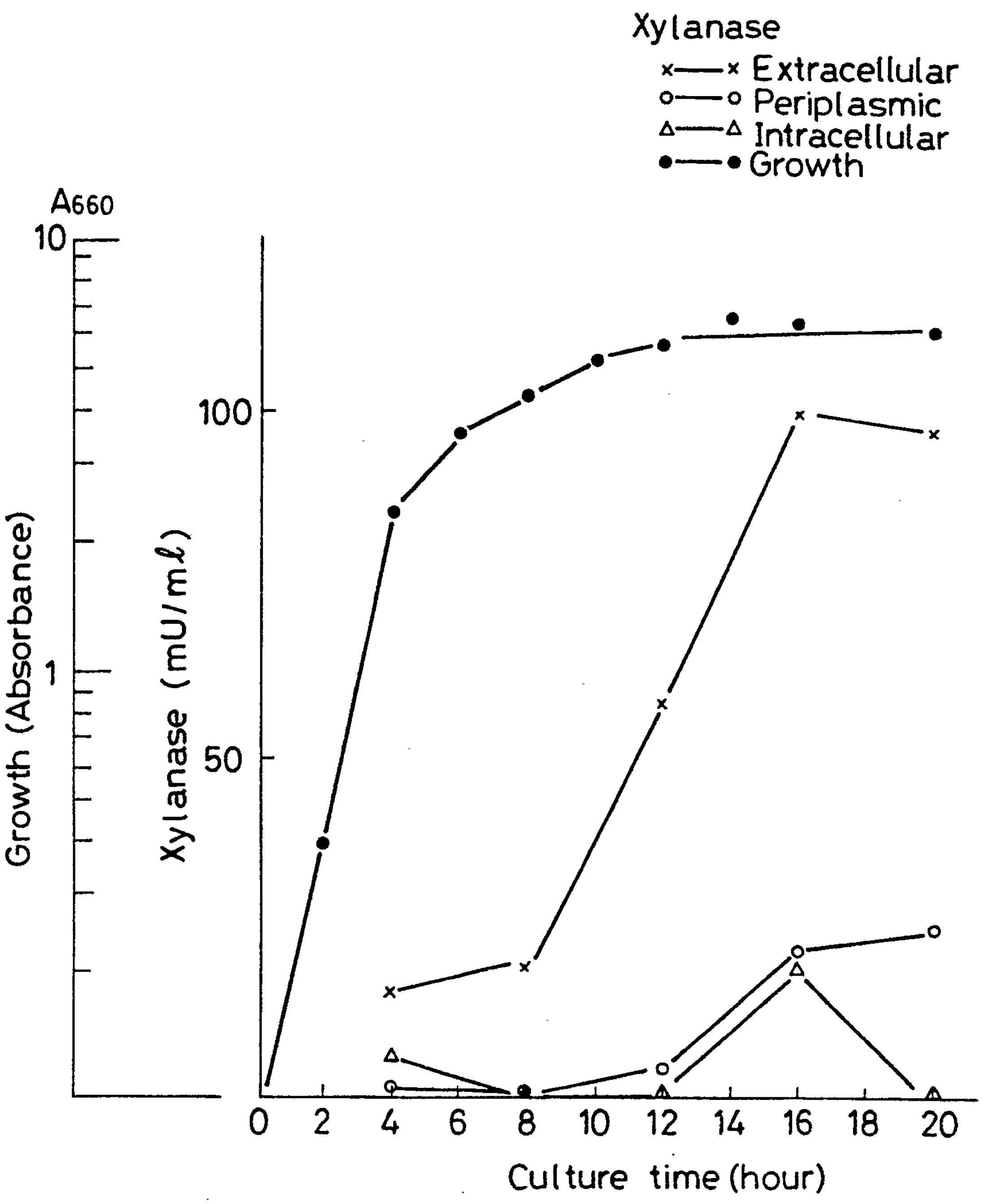
Xylanase
×——× Extracellular
○——○ Periplasmic
△——△ Intracellular
●——● Growth
A660
10
1
Growth (Absorbance)
100
50
Xylanase (mU/mℓ)
0 2 4 6 8 10 12 14 16 18 20
Culture time (hour)

FIG.6

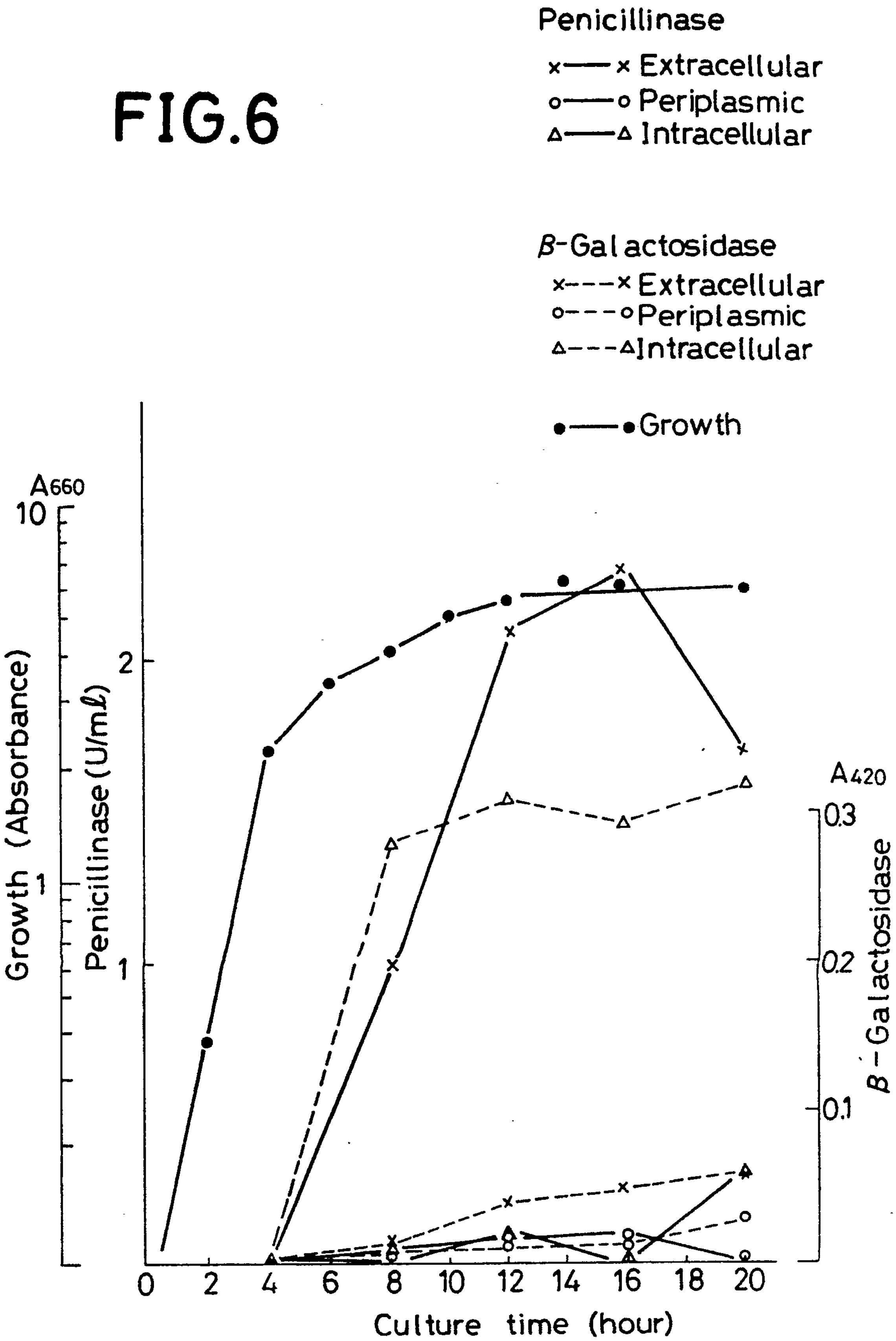
Penicillinase
x——x Extracellular
o——o Periplasmic
Δ——Δ Intracellular
β-Galactosidase
x---x Extracellular
o---o Periplasmic
Δ---Δ Intracellular
●——● Growth
A660
10
2
1
Growth (Absorbance)
Penicillinase (U/mℓ)
A420
0.3
0.2
0.1
β-Galactosidase
0
2
4
6
8
10
12
14
16
18
20
Culture time (hour)